# NEW EUROPEAN PATENT SPECIFICATION

(11) **EP 0 437 488 B2**
(45) Date of publication and mention of the opposition decision: **19.12.2001**
(45) Mention of the grant of the patent: 14.01.1998
(21) Application number: 89911319.5
(22) Date of filing: 05.10.1989
(51) Int. Cl.: G01N 33/574, C12N 5/08, A01K 67/027

(54) **ANIMAL MODEL FOR HUMAN DISEASE**
TIERMODELL FÜR MENSCHLICHE KRANKHEIT
MODELE ANIMAL POUR MALADIES HUMAINES

(30) Priority: 05.10.1988 US 253990
(43) Date of publication of application: 24.07.1991
(73) Proprietor: ANTICANCER, INC., San Diego, CA 92111 (US)
(72) Inventor: MONOSOV, Ann, San Diego, CA 92122 (US); RAY, Gustav, San Diego, CA 92120 (US)
(74) Representative: Fisher, Adrian John
(86) International application number: US8904414
(87) International publication number: WO9004017

(56) References cited:
- CANCER RESEARCH, vol. 46, 01 April 1986, Washington, DC (US); R. GIAVAZZI et al., pp. 1928-1933/
- PROCEEDINGS OF THE NATL. ACADEMY OF SCIENCES USA, vol. 88, no. 20, 15 October 1991, Washington, DC (US); X. FU et al., pp. 9345-9349/
- SEMINARS IN ONCOLOGY, vol. 8, December 1981, Orlando, FL (US); OVEJERA et al., pp. 386-393/
- EXPERIMENTAL CELL BIOLOGY, vol. 50, 1982, Basel (CH); WANG et al., pp. 330-331/
- CANCER RESEARCH, vol. 42, March 1982, Baltimore, MD (US); WHITE et al., pp. 906-912/
- CANCER RESEARCH, vol. 48, December 1988, Baltimore, MD (US); MORIKAWA et al., pp. 6863-6871/
- JOURNAL OF THE NATL. CANCER INSTITUTE, vol. 78, February 1987, BEthesda, MD (US); NAITO et al., pp. 377-385/
- CANCER RESEARCH, vol. 46, August 1986, Baltimore, MD (US); NAITO et al., pp. 4109-4115/
- Shapiro W R et al. (1979) J. Natl.Cancer Inst. 62 : 447-453
- Fidler I J (1986) Cancer and Metastasis Reviews 5: 29-49
- Kyriazis A P et al.(1981) Cancer Research 41 : 3995-4000
- Morikawa K et al. (1988) Cancer Research 48 : 1943-1948

## Description

### BACKGROUND OF THE INVENTION

The present invention relates to an animal model for human neoplastic disease. More particularly, the invention relates to an animal model having neoplastic tissue, obtained from a human organ, implanted into the corresponding organ of the animal.

There has long been a need for a representative animal model for human neoplastic disease. Such a model could serve many purposes. For example, it could be used to study the progression of neoplastic disease in humans and assist in finding appropriate treatment forms. Such a model could also be used to test the efficacy of proposed new anti-neoplastic agents. Additionally, it could be employed in individualized chemosensitivity testing of a cancer patient's tumors. The existence of such an animal model would make drug screening, testing and evaluation much more efficient and much less costly.

Some previous attempts at generating animal models for human neoplastic disease employed transplantable animal tumors. These were tumors that had developed in rodents and had been transplanted from animal to animal, usually in inbred populations. Other animal tumor models were generated by inducing tumors in the animals by means of various agents that were carcinogenic, at least in the animal system. Still other animal tumor models were rodents containing spontaneously-occurring tumors. These rodent models, however, frequently responded to chemotherapeutic agents very differently than human subjects receiving the same agent.

Another animal tumor model that developed starting some twenty years ago utilized mice without a thymus. These animals were deficient in cellular and therefore lost their ability to reject foreign transplant tissue. The mice, for reasons not clearly understood, were essentially lacking in hair and came to be called "nude" or "athymic" mice.

It was found that human tumors often grew when implanted subcutaneously under the skin of these nude mice. However, the take rate or frequency with which such human tumor tissue actually formed a tumor in the mouse varied depending on the individual donor and the tumor type. In these models, tumors that took, often grew to a great extent at the site of implant and rarely metastasized, even if the original tumor had been highly metastatic in the donor. Accordingly, the subcutaneous nude mouse tumor model, although better than the previously described rodent model, still had a substantial drawback, i.e. the subcutaneous transplant lacked the ability to metastasize.

Naito *et al.,* Cancer Research 46, 4109 to 4115 (1986), Wang *et al.,* Experimental Cell Biology, 50, 330 to 331 (1982) and Ovejera *et al.,* Seminars in Oncology, 8, 386 to 393 (1981) disclose the implantation of disag-gregated human neoplastic tissue in the form of cell suspensions in animal models.

To fulfil the need for an animal model for human neoplastic disease which is without the above-mentioned deficiencies, the present invention discloses a new animal model which has the ability to truly mimic the progression of neoplastic disease as it occurs in humans.

### SUMMARY AND OBJECT OF THE INVENTION

It is the primary object of the present invention to provide an improved animal model for human neoplastic disease.

In accordance with the present invention, a novel animal model for human neoplastic disease is provided having neoplastic tissue obtained from a human organ, as defined in claim 1, implanted into the corresponding organ of the animal and having sufficient immunodeficiency to allow the implanted tissue to grow and metastasize.

### DETAILED DESCRIPTION OF THE INVENTION

The animal model of the present invention is generated by implantation of human neoplastic tissue into a laboratory animal having sufficient immunodeficiency to allow the implanted tissue to grow and metastasize. A particularly suitable laboratory animal for use herein is a strain of mice that has no T-cell immunity. These mice, commonly referred to as nude mice or athymic mice, are readily available and may be obtained commercially from Charles River Laboratories, Inc., Wilmington, Massachusetts (Catalog identification: Crl:nu/nu (CD-1)BR, homozygous 28-42 days old).

The placement of neoplastic tissue in an immunodeficient laboratory animal according to the present invention is carried out by means of orthotopic implantation. This refers to an implant or graft transferred to a position formerly occupied by tissue of the same kind. In the present invention, the terminology orthotopic implantation is used to refer to the grafting of human neoplastic tumor tissue from a human organ into the corresponding organ of an immunodeficient laboratory animal. The human neoplastic tissue utilized herein comprises tissue from fresh surgical specimens which are pathologically diagnosed tumors occurring in human kidney, liver, stomach, pancreas, colon, breast, prostate, lung and testis. Such tumors include carcinomas as well as sarcomas and implantation thereof as carried out herein encompasses all stages, grades and types of tumors.

Prior to implantation, the human neoplastic tissue is maintained by placing in a suitable nutrient medium, such as Eagle's minimum essential medium containing ten percent fetal calf serum and a suitable antibiotic, such as gentamycin. The medium containing the tissue is then cooled to approximately 4 degrees C. The tissue can be maintained in this manner for approximately twenty-four hours.

A selected tissue specimen is prepared for implantation by forming into a mass of suitable size for insertion into a suitably prepared cavity in the selected organ. The specimen size may vary from about 0.1 x 0.5 cm to about 0.2 x 0.6 cm. The technique used to form a specimen of suitable size comprises teasing the tissue to size by pulling into pieces of the desired size with forceps orthe like.

Microsurgical instruments typically used to carry out tissue implantation according to the present invention include a castrovijeo needle holder, jeweler's forceps (straight and curved), iris forceps, iris scissors and straight and curved tissue forceps, including one each with teeth and one each without teeth.

Prior to implantation of neoplastic tissue, the selected immunodeficient animal is anesthetized with a suitable anesthetic. The implantations of all organ tissue mentioned herein, except lung tissue, is conveniently accomplished by conventional anesthesia using ethyl ether. Where lung tissue is implanted, pentobarbital is used as the anesthetic.

Implantation of tissue from a hepatoma or tumor from a human liver is carried out utilizing the caudal lobe of the recipient animal's liver as the implantation site. Several loose sutures are placed over the lobe and an incision is made longitudinally to accommodate a tumor mass of approximately 0.2 x 0.5 cm in size. After placement of the tumor in the incision, the sutures are pulled snugly over the tumor in order to secure it is place.

The process of implantation of tissue from a human pancreatic tumor is carried out by making an incision in the recipient animal's pancreas at the head of the organ nearthe duodenum. Care is exercised to select an avascular area. An incision is made in the selected area and a tumor mass of approximately 0.5 x 0.2 cm is implanted in a manner identical to that described in the preceding paragraph. Tissue from all stages and all grades of pancreatic carcinoma may be implanted in this manner.

The implantation of tissue from a human mammary carcinoma is carried out by surgically forming a pocket on the breast of the recipient female animal. The pocket is preferably sufficient to accommodate a tumor mass approximately 0.2 x 0.5 cm in size. After placement of the tumor in the pocket, the pocket is closed with a suture. All stages and grades of mammary carcinoma may be implanted in this manner.

Implantation of tissue from a human prostatic carcinoma into the prostate of the recipient animal is carried out by surgically forming an opening in the prostate and then placing a tissue specimen of approximately 0.2 x 0.3 cm in size in the lumen. After placement of the tissue specimen, the opening is closed with appropriate sutures. Two additional sutures are placed at the neck of the prostate so that the implanted tissue will not be able to migrate from the original site.

Implantation of tissue from a human testicular carcinoma into the testis of a recipient animal is carried out by penetrating the testis along the longitudinal axis with a number-18 gauge needle and injecting a tumor mass of approximately 0.1 x 0.5 cm in size through the needle. When the end of the tumor specimen is visible at the time of the needle, the needle is gently withdrawn while visible tumor tissue is held in place with forceps. The hole made by the needle is then closed with a single suture.

In preparation for implantation of neoplastic lung tissue into the lungs of the recipient animal, a tracheotomy is performed and silastic tubing is intubated. Thereafter either of the following implantation procedures may be employed:
(1) the tracheotomy tubing is advanced to reach either lung lobe(s); asmall elongated (2x6 mm) tumor mass is injected through the tubing; and the tubing is then removed and the tracheal wound is closed with a suture; or
(2) precautionary tubing is inserted into the trachea; a small stab wound is made on the right chest to bring up a lobe of the right lung which plugs the thoracic cavity thereby preventing collapse of the lung; the lung lobe is gently clamped at the base and two ligatures are loosely placed on the lung; an incision is made on the lung, a tumor of approximately 0.2 x 0.5 cm is imbedded therein and the ligatures are snugly tied; the lung lobe in placed back into the thoracic cavity and the wound is closed.

Tissue from all stages and grades of small cell and non-small cell lung carcinomas may be implanted by either of the above-described procedures.

The animal models of the present invention are particularly useful in studying the progression of human neoplastic disease. These studies, in combination with other clinical testing modalities such as diagnostic imaging, help in the selection of the most appropriate form of treatment

For example, when an animal model of the present invention is subjected to tumor imaging, the clinician is allowed to identify both primary and secondary sites of tumor growth and to estimate the overall burden of the tumor on the animal. Tumor imaging is conventionally carried out by injecting the animal with a labeled antitumor antibody such as an antibody labeled with a radioactive isotope; allowing the antibody time to localize within the tumor; and then scanning the animal using a radiation detector. When a computer is used to compile an image of the radioactivity detected in the animal's body, the computer can color code the image according to the intensity of the radiation. Zones of high radioactivity in regions of the body not expected to accumulate the antibody or its metabolites indicate the possible presence of tumors.

The animal models of the present invention can also be used to screen new anti-neoplastic agents to determine the ability of such agents to affect tumors at the primary site and also at distant metastatic sites or to prevent distant metastases from occurring. The models will be also useful for individualized chemosensitivity testing of a cancer patient's tumors.

Additionally, the animal models of the present invention are useful in studying the effects of mitrution on the progression of human neoplastic disease. These studies can be particularly significant in view of the demonstrated impact of various deficiencies on healthy subjects.

### EXAMPLE I

Fresh surgical specimens of tissue from a tumor excised from a human kidney were transplanted into the kidneys of five animal recipients. The tissue specimens, which were pathologically diagnosed as renal cell carcinoma, were prepared to size by the teasing procedures described earlier.

Five athymic nude mice age four (4) to six (6) weeks were selected as the animal recipients for the implants. In preparation for surgery, the mice were anesthetized with ether. An incision was made in each animal to access the kidney. A wedge shaped cavity was formed by excision of the renal cortex of each recipient kidney and a mass of tumor tissue of approximately 0.5 x 0.2 cm was placed in the defected cavity. A mattress suture was then employed to secure the implant in place.

The five mice of this example are still alive six months later. Approximately one month following implantation of the tissue, the mice were surgically opened and the implanted tumors were observed. In each case, the tumor was found to have taken. This means that the implanted neoplastic tissue had invaded adjacent tissue. Histological analysis was performed on the tissue implants at this time. Such analysis comprised removing tissue samples from each animal and comparing the samples with a tissue sample from the tissue donor.

Preparation of the tissue samples for histological analysis was carried out by (1) fixing the sample in formalin; (2) embedding the fixed sample in paraffin; (3) preparing 5 micron sections of the fixed, embedded sample; (4) staining the sections with hematoxylin and eosin; and (5) microscopically observing the tissue structure in each section.

Histological analysis revealed that the tissue in the recipient animals (1) preserved its architecture and tissue type and (2) mimicked progression of the disease in the human donor.

### EXAMPLE II

Specimens of human tissue excised from the stomach and pathologically diagnosed as gastric carcinoma were prepared to size by the teasing procedure described earlier.

Five athymic nude mice age four (4) to six (6) weeks were selected as the animal recipients for the implants. In preparation for surgery, the mice were anesthetized with ether.

Each anesthetized mouse was opened to provide access to the stomach. An incision was made in the stomach wall using a number 11 scalpel taking care not to penetrate the mucosal layer. A pocket was formed large enough to receive a tumor mass of about 0.5x0.2 cm. A tumor of approximately this size was selected and inserted into the pocket and the incision was closed using a 7-0 suture.

The five mice of this example have survived for about three (3) to four (4) months and otherwise appear healthy. Subsequent surgical opening of the stomach of these mice has verified that the tumors have taken.

### EXAMPLE III

Specimens of human tissue removed from a human colon and pathologically diagnosed as colon carcinoma were prepared to size by the teasing procedure described earlier. Five athymic nude mice, age four (4) to six (6) weeks were selected as the animal recipients for the implants. In preparation for surgery, the mice were anesthetized with ether.

Each anesthetized mouse was opened to provide access to the colon. A pocket of cavity was surgically formed in the seromuscular layer with care exercised not to enter the lumen. A selected tumor mass of approximately 0.5 x 0.2 cm was inserted into the pocket which was then closed with a suture.

Four of the five mice which underwent this implant surgery have survived for three to four months and appear to be in good health.

Approximately one month following tissue implantation, the mice were surgically opened and the tumors were observed to have taken. None of the tumors appeared not have metastasized to other organs at this time.

Although the foregoing invention has been described in some detail by way of illustration and example for purposes of clarity of understanding, it will be obvious that certain changes and modifications may be practiced within the scope of the appended claims.

## Claims

1. A non-human animal model for human neoplastic disease, said animal model having neoplastic cells obtained from a human liver, kidney, stomach, pancreas, colon, breast, prostate, lung or testis implanted into the corresponding organ of said animal, and having sufficient immunodeficiency to allow said implanted neoplastic tissue to grow and metastasize, **characterised in that** said cells are in the form of an intact mass of neoplastic tissue.

2. An animal model according to claim 1 wherein said animal is an athymic mouse.

3. An athymic mouse model according to claim 2 wherein said neoplastic tissue is obtained from human kidney.

4. An athymic mouse model according to claim 3 wherein said human neoplastic kidney tissue is implanted in the renal cortex of the kidney of the mouse.

5. An athymic mouse model according to claim 2 wherein said neoplastic tissue is obtained from human stomach.

6. An athymic mouse model according to claim 5 wherein said human neoplastic stomach tissue is implanted in the stomach of the mouse between the inner mucosal lining of the stomach and the outer peritoneal coat of the stomach.

7. An athymic mouse model according to claim 2 wherein said neoplastic tissue is obtained from human colon.

8. A female athymic mouse model according to claim 2 wherein said neoplastic tissue is obtained from a female human breast.

## Patentansprüche

1. Nicht-humanes Tiermodell für eine humane neoplastische Erkrankung, wobei dieses Tiermodell neoplastische Zellen, die aus einer humanen Leber, Niere, Pankreas, Dickdarm, Brust, Prostata, Lunge oder Hoden erhalten wurden, in das entsprechende Organ des Tieres eingepflanzt enthält und eine ausreichende Immundefizienz hat, um dem implantierten neoplastischen Gewebe Wachstum und Metastasierung zu erlauben, **dadurch gekennzeichnet, daß** die Zellen in der Form einer intakten Masse von neoplastischem Gewebe vorliegen.

2. Tiermodell nach Anspruch 1, worin das genannte Tier eine athymische Maus ist.

3. Athymisches Mausmodell nach Anspruch 2, worin das genannte humane neoplastische Gewebe aus humaner Niere gewonnen wurde.

4. Athymisches Mausmodell nach Anspruch 3, worin das genannte humane neoplastische Nierengewebe in den Renalcortex der Niere der Maus implantiert ist.

5. Athymisches Mausmodell nach Anspruch 2, worin das genannte neoplastische Gewebe aus humanem Magen gewonnen wurde.

6. Athymisches Mausmodell nach Anspruch 5, worin das genannte humane neoplastische Magengewebe in den Magen der Maus zwischen der inneren Schleimhautauskleidung des Magens und dem äußeren peritonealen Mantel des Magens implantiert ist.

7. Athymisches Mausmodell nach Anspruch 2, worin das genannte neoplastische Gewebe aus humanem Dickdarm gewonnen wurde.

8. Weibliches athymisches Mausmodell nach Anspruch 2, worin das genannte neoplastische Gewebe aus einer humanen weiblichen Brust gewonnen wurde.

## Revendications

1. Modèle animal non humain pour maladies humaines néoplasiques, ledit modèle animal ayant des cellules néoplasiques obtenues à partir d'un foie, d'un rein, d'un estomac, d'un pancréas, d'un côlon, d'un sein, d'une prostate, d'un poumon ou des testicules, humain implanté à l'intérieur de l'organe correspondant dudit animal, et ayant une immunodéficience suffisante pour permettre audit tissu néoplasique implanté de pousser et de métastaser, **caractérisé en ce que** lesdites cellules sont sous forme d'une masse intacte de tissu néoplasique.

2. Modèle animal selon la revendication 1, **caractérisé en ce que** ledit animal est une souris sans thymus.

3. Modèle souris sans thymus selon la revendication 2, **caractérisé en ce que** ledit tissu néoplasique est obtenu à partir du rein humain.

4. Modèle souris sans thymus selon la revendication 3, **caractérisé en ce que** ledit tissu néoplasique du rein humain est implanté dans le cortex rénal du rein de la souris.

5. Modèle souris sans thymus selon la revendication 2, **caractérisé en ce que** ledit tissu néoplasique est obtenu à partir de l'estomac humain.

6. Modèle souris sans thymus selon la revendication 5, **caractérisé en ce que** ledit tissu néoplasique de l'estomac humain est implanté dans l'estomac de la souris entre le revêtement intérieur de la muqueuse de l'estomac et le revêtement extérieur du péritoine de l'estomac.

7. Modèle souris sans thymus selon la revendication 2, **caractérisé en ce que** ledit tissu néoplasique est obtenu à partir du côlon humain.

8. Modèle souris sans thymus selon la revendication 2, **caractérisé en ce que** ledit tissu néoplasique est obtenu à partir du sein d'une femme.
